# EUROPEAN PATENT APPLICATION

(11) **EP 1 323 825 A2**
(43) Date of publication of application: **02.07.2003**
(21) Application number: 02425681.0
(22) Date of filing: 08.11.2002
(51) Int. Cl.: C12N 15/53, C12N 15/82, C12N 5/10, A01H 5/00, A01H 5/10

(54) **Recombinant plants and DNA constructs**

(30) Priority: 09.11.2001 IT RM20010670
(71) Applicant: ENEA ENTE PER LE NUOVE TECNOLOGIE, L'ENERGIA E L'AMBIENTE, 00196 Roma (IT); Biogen S.r.l., 00155 Roma (IT)
(72) Inventor: Giuliano, Giovanni, 00196 Roma (IT); Rosati, Carlo, 00196 Roma (IT); Dharmapuri, Sridhar, 00196 Roma (IT); Pallara, Patrizia, 00196 Roma (IT); Camara, Bilal, CNRS, Plant Molecular, 67084 Strasbourg Cedex (FR)
(74) Representative: Taliercio, Antonio

(57) **Abstract**

A process of increasing the metabolites of carotene content and in particular the carotenoid such as zeaxanthin content of a plant, said process comprising upregulating a gene which encodes either lycopene β-cyclase and β-carotene hydroxylase activity. Constructs which can be used in the process are also described and claimed.

## Description

The present invention relates to plant gene expression cassettes which can be used to produce recombinant plants which express high levels of metabolites of carotene, more in detail of carotenoids, including oxygenated carotenoids, to DNA constructs and to plants incorporating them.

Carotenoids comprise a large multifunctional group of terpenoid pigments which are essential components of the photosynthetic membranes in all plants, algae and cyanobacteria. They play an important role in human nutrition, particularly on account of their pro vitamin A activity. β-carotene is the dietary precursor of Vitamin A and its deficiency (VAD) leads to xerophthalmia - the principal cause of childhood blindness in developing countries. Avitaminosis is also the leading cause of child mortality in these regions as a result of greater susceptibility to respiratory infections, diarrhoea and measles. Other carotenoids such as lycopene, lutein and zeaxanthin are dietary anti-oxidants and are extensively used as industrially safe colorants of food and cosmetic formulations. High plasma levels of lycopene have been known to be associated with a decreased incidence of prostate cancer. The carotenoid xanthophylls, lutein and zeaxanthin, accumulate in the eye lens and macular region of the retina and contribute greatly to a reduction in the risk of degenerative eye diseases like cataract and macular degeneration. Studies have shown that with adequate supplementation of dietary Vitamin A, infant mortality risk in pregnant women is reduced by 50% and VAD - related disorders in children by 33%. However, as supplements are difficult and expensive to administer effectively, the alternative is the increase of food crops in β-carotene and zeaxanthin.

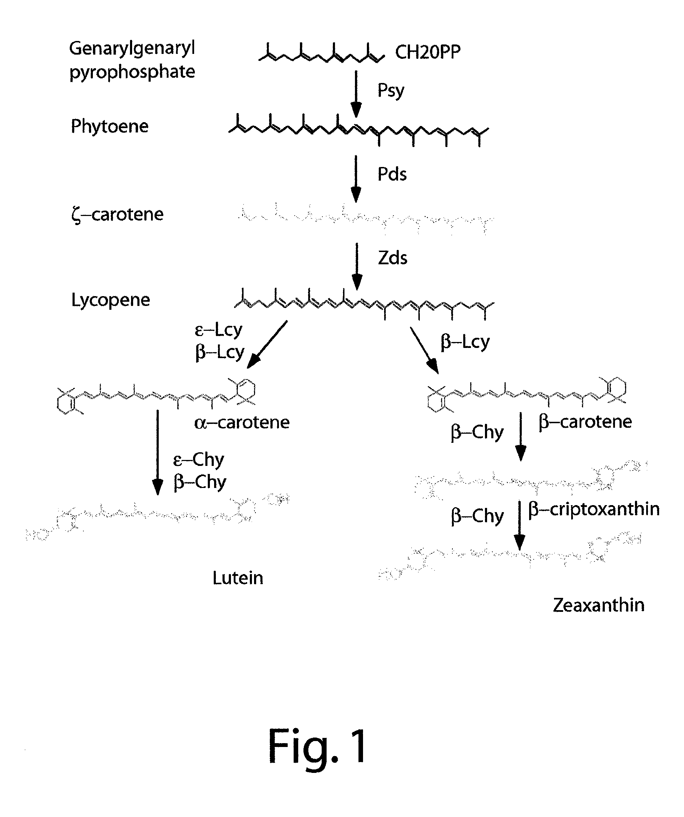

Carotenoids are synthesized in the plastids of plants. The first dedicated step is the dimerisation of geranylgeranyl pyrophosphate (GGPP) to form phytoene. Subsequent desaturations by phytoene desaturase and by ξ-carotene desaturase result in the production of the linear carotenoid, lycopene . This is a key branch-point in the pathway and is illustrated hereinafter in Figure 1. Lycopene is the precursor for the formation of most cyclic carotenoids through further modifications catalyzed by cyclases, hydroxylases, ketolases and epoxidases. Unsubstituted β-ionone rings are introduced at both ends of the lycopene molecule by the enzyme lycopene β-cyclase (such as β-Lcy) to form the first cyclic carotenoid, β-carotene. A competing ε-cyclase (such as ε-Lcy) enzyme introduces ε-ionone rings to make ∝-carotene. Subsequent mono-and di-hydroxylation of the β-ionone rings by β-carotene hydroxylase (β-Chy) leads to the formation of the xanthophylls, β-cryptoxanthin and zeaxanthin respectively (Figure 1). All enzymes are encoded by nuclear genes and a number of them have been cloned in recent years (Cunningham, F. X. et al. (1998) *Ann. Rev. Plant Physiol. Plant Mol. Biol.* 49, 557-583; Harker, M. et al. (1998) *Meth. Enzymol.* 297, 256-269).

Among crop plants, tomato (*Lycopersicon esculentum)* is one of the most productive in terms of carotenoids produced per cultivated area. It is also very convenient to study due to the distinct colour changes (primarily as a function of carotenoid accumulation) in its fruit during ripening. Our understanding of the carotenoid biosynthetic pathway in tomato has made great progress in the last few years. Many single mutations that affect carotenoid accumulation in the fruit such as *yellow flesh, Delta, high-pigment-2, old gold* and *Beta* have been isolated and characterized. Most of the genes involved in carotenoid biosynthetic pathway have been cloned and their regulation extensively studied. In the initial stages of development, green tomato fruits have a carotenoid content similar to leaves essentially, β-carotene, lutein and violaxanthin with low levels of lycopene and trace amounts of other xanthophylls. At the breaker stage of ripening, lycopene accumulation increases dramatically (up to 90% of total carotenoids) and the fruit turns red. At this stage, the genes controlling lycopene synthesis such as *Psy1* and *Pds* (encoding the fruit specific phytoene synthase and phytoene desaturase respectively) are up-regulated (Pecker, I et al. (1992) *Proc. Natl. Acad. Sci. USA* 89, 4962-4966; Giuliano, G et al. (1993) *Plant Cell* 5, 379-387). Genes encoding lycopene cyclases like *β-Lcy* and *ε-Lcy* are down-regulated resulting in very low levels of β-carotene and, consequently, xanthophylls (5-10% and 1-4% of total carotenoids respectively) in ripe tomato fruits (Ronen, G., et al. (1999) *Plant J.* 17, 341-351; Pecker, I et al. (1996) *Plant Mol. Biol.*30, 807-819). By introducing 1-3 biosynthetic steps, this large pool of lycopene can be converted into high value - added compounds like β-carotene, zeaxanthin and lutein can transform tomato into a bio-factory for the production of carotenoid metabolites.

Fruits of the *B* (*Beta*) genotype typically have a β-carotene content of 50% or above. The *B* gene has been shown to encode an alternative *β-Lcy* and is suggested to be the major β-carotene synthesizing enzyme in tomato (Ronen, G., et al. (2000) *Proc. Natl. Acad. Sci. USA* 97, 11102-11107). This genotype could be particularly useful for production of carotenoid metabolites downstream of β-carotene.

Production of important dietary supplements such as β-carotene, anti-oxidants such as zeaxanthin and high value - added compounds for industrial use like astaxanthin has been one of the goals of metabolic engineering. The last named compound is a non-plant carotenoid that has been engineered in the nectary of tobacco (Mann, V et al. (2000) *Nat. Biotechnol.* 18, 888-892). However, the challenge for metabolic engineering of carotenoids is their overproduction in important crops like tomato.

The metabolic engineering of lycopene and β-carotene in tomato fruits by over-expressing the *β-Lcy* gene in antisense and sense orientations, respectively, has been reported previously (Rosati, C., et al. (2000) *Plant J.* 24, 413-420).

The applicants have now found that by upregulating certain genes, the levels of carotenes and metabolites and in particular β-carotene and xanthophylls present in plants can be increased.

The present invention provides a process for increasing carotene's metabolite content of a plant, particularly oxygenated carotenoids, said process comprising upregulating at least genes encoding carotene hydroxylase activity (e.g. β-carotene hydroxylase activity), preferably genes encoding lycopene cyclase (e.g. lycopene β-cyclase) and carotene hydroxylase activity (e.g. β-carotene hydroxylase activity). In particular, the process increases the zeaxanthin content of a plant such as a crop plant.

Upregulation of the endogenous genes may be achieved using processes outlined below. Preferably however, upregulation will be effected by transforming the plant so that the genome contains more copies of a gene, which encodes an enzyme having the desired activity.

In particular, the plant is transformed with both genes which encode lycopene β-cyclase and β carotene hydroxylase activity both of which are upregulated.

In a further aspect, the present invention provides a plant gene expression cassette, which comprises a first DNA sequence which is capable of producing over-expression of a gene which encodes a protein having lycopene β-cyclase activity and a second DNA sequence which is capable of producing over-expression of a gene which encodes a protein having β-carotene hydroxylase activity.

In the context of the present application, the term "over-expression" means that the level of expression of the specified gene and the enzyme for which it encodes, is increased as compared to the levels of that gene expressed in a wild-type or unmutated form of the plant cell.

Overexpression can be achieved in a number of ways. For example, genes can be upregulated by transforming the plant such that endogenous lycopene β-cyclase and β carotene hydroxylase genes are overexpressed. This may be achieved by introducing into the plant by transformation, a regulatory gene such as a transcription factor, which acts on the endogenous gene sequence so as to enhance transcription from the promoter of the endogenous gene.

Conveniently, however, the construct comprises a sequence which encodes a protein having lycopene β-cyclase activity and/or a protein having β-carotene hydroxylase activity, each being under the control of a plant promoter sequence.

The expression "plant promoter sequence" as used herein refers to promoters which are active in allowing expression of genes in plants.

Introduction of at least one further copy of the coding sequence into the plant in this way, in a manner in which it is expressed in the plant and in particular in the fruit, ensures that lycopene β-cyclase and/or β-carotene hydroxylase or an active fragment thereof are overexpressed in the crop part such as the fruits of the plants.

Suitable proteins having lycopene β-cyclase activity include wild-type enzymes, which may be homologous or heterologous to the plants to be transformed, active fragments of these, or variants of any of these. Similarly proteins having β-carotene hydroxylase activity will include wild-type enzymes, which may be homologous or heterologous to the plants to be transformed, active fragments of these, or variants of either of these. Heterologous genes may be derived from various organisms including other plants or microorganisms, such as bacteria and in particular cyanobacteria.

The term "variants" as used herein includes allelic variants, and genes which have some degree of similarity of structure to the gene in question, and encode proteins with a similar function, but which are derived from organisms, including other plant species or micro-organisms, as well as chimeric genes derived from more than one of these. Suitably the level of structural similarity between the basic gene and the homologue is at least 50%, for example, at least 70%, preferably at least 80%.

Generally speaking, homologues or homologous genes may be identified either by comparison of sequence data using any of the known homology programmes, for example, those exemplified below, or by conducting hybridisation experiments.

The hybridisation experiments will identify genes or DNA which hybridise to the basic DNA. Preferably, such hybridisation occurs at, or between, low and high stringency conditions. In general terms, low stringency conditions can be defined as 3 x SCC at about ambient temperature to about 65°C, and high stringency conditions as 0.1 x SSC at about 65°C. SSC is the name of a buffer of 0.15M NaCI, 0.015M trisodium citrate. 3 x SSC is three times as strong as SSC and so on.

The term "variant" also includes DNA sequences which encode sequences of amino acids which differ from the base sequence from which they are derived in that one or more amino acids within the sequence are substituted for other amino acids, but which retain similar biological activity. Amino acid substitutions may be regarded as "conservative" where an amino acid is replaced with a different amino acid with broadly similar properties. Non-conservative substitutions are where amino acids are replaced with amino acids of a different type. Broadly speaking, fewer non-conservative substitutions will be possible without altering the biological activity of the polypeptide. Suitably variants will have levels of similarity of at least 60%, preferably at least 75%, and more preferably at least 90% to the base sequence.

As used herein the term "level of similarity" is used to denote sequences which when aligned have similar (identical or conservatively replaced) amino acids in like positions or regions, where identical or conservatively replaced amino acids are those which do not alter the activity or function of the protein as compared to the starting protein. For example, two amino acid sequences with at least 85% similarity to each other have at least 85% similar (identical or conservatively replaced amino residues) in a like position when aligned optimally allowing for up to 3 gaps, with the *proviso* that in respect of the gaps a total of not more than 15 amino acid resides is affected. The degree of similarity may be determined using processes well known in the art (see, for example, Wilbur, W.J. and Lipman, D.J. "Rapid Similarity Searches of Nucleic Acid and Protein Data Banks." Proceedings of the National Academy of Sciences USA 80, 726-730 (1983) and Myers E.and Miller W. "Optimal Alignments in Linear Space". Comput. Appl. Biosci. 4:11-17(1988)). One programme which may be used in determining the degree of similarity is the MegAlign Lipman-Pearson one pair process (using default parameters) which can be obtained from DNAstar Inc, 1228, Selfpark Street, Madison, Wisconsin, 53715, USA as part of the Lasergene system.

Variants may be obtained synthetically, for example using gene shuffling techniques.

Preferably, however the proteins having lycopene β-cyclase activity and β-carotene hydroxylase activity are wild-type lycopene β-cyclase enzymes and wild-type β-carotene hydroxylase enzymes respectively. Therefore, the first and second DNA sequences suitably comprise genes which encode a lycopene β-cyclase and β-carotene hydroxylase respectively.

It may be preferable to use a heterologous gene, especially where the gene, or RNA or protein is regulated differently to that of the homologous gene. For example, plant genes may be suitably employed in different plants. In addition, use of a heterologous gene may reduce problems associated with endo-regulation and/or gene silencing. If a bacterial gene is used in the process of the invention, it is suitably accompanied by a plastid transit peptide to direct the protein to the plastid compartment. An example is the plastid transit peptide from the pea RUBISCO small sub-unit.

Thus in a particular embodiment, the invention provides a plant gene expression cassette comprising
(i) a first DNA sequence which encodes a protein having lycopene β-cyclase activity under the control of a first plant promoter, and
(ii) a second DNA sequence which encodes a protein having β-carotene hydroxylase activity under the control of a second plant promoter.

The first and second plant promoters may be the same or different, provided they are active at the same time in the plant cells. In particular, the promoters may be constitutive, inducible, tissue-specific, development specific or even be under the control of "gene switches" such as those illustrated for example in WO 93/21334 and WO 96/37609. In particular, the promoters should be selected so that the first and second DNA sequences are expressed in the crop parts of the plant such as the fruit as this will ensure that that the beneficial effects in terms of enhanced nutrient content is fulfilled.

Preferably, the first and second promoters are promoters which are specific for a particular target tissue of the plant, such as the crop tissue, as this will minimise the genetic and metabolic burden on the plant overall.

The term "crop tissue" used herein refers to that part of the plant which is intended for use as a foodstuff or feed, fibres (such as cotton fibres), or flowers. The crop may be a horticultural or field crop. Thus in tomatoes, the crop tissue will be the fruit and the tissue specific promoter employed will be a fruit specific promoter. In other crops, this may be the leaf tissue (for example, lettuce, cabbage, spinach and Lemna or pondweed), seed tissue (e.g. in grain such as maize or wheat) root tissue (in vegetables such as carrots, and potatoes) or flowers (such as marigolds) and the promoters are selected accordingly.

Suitable tissue-specific promoters will depend upon the plant being considered and the particular tissue being targetted (e.g. fruit or leaf).

Examples of tomato fruit promoters are the Pds, E8, tomato polyglacturonase, tomato invertase, *Lycopersicon pimpinellifollium* invertase, tomato ACC oxidase, and pepper fibrillin promoters. Examples of cereal grain promoters are the rice glutellin promoter, wheat HMW glutenin promoter, maize ESR (Bonello *et al.* 2000) and Betl 1 promoters (Hueros *et al*.,1999), the END1 promoter from barley (Doan *et al.,* 1996 ) the wheat high molecular glutenin (REF required), rice PCNA (expressed in proliferating cells ie. endosperm and meristems) and blz2 (transcription factor). Examples of cereal leaf promoters are the maize MS8-15, maize chlorophyll a/b binding protein (Cab) (Sullivan et al., 1989), rice RTBV promoter and the maize phosphoenolpyruvate carboxylase promoter.

Other crop tissue-specific promoters can be obtained by conventional processes. For example, differential screening of DNA libraries from the desired crop tissue as compared to other plant tissue can be used to identify genes which are expressed only in the crop tissue. Such processes are illustrated with respect to the isolation of germination specific promoters in for example International Patent Publication No.97/35983.

In outline, a range of partial clones are amplified by reverse transcriptase polymerase chain reaction (RT-PCR) on RNA from the target crop tissue. Preliminary assessment of the clones' expression in the crop tissue can be made by northern blotting. More detailed northern blot experiments on selected clones may be carried out, for example assess their time course of expression. A cDNA library is then constructed from tissues showing a high expression of these clones, followed by screening of a genomic library to subclone the promoter areas. Final assessment of the spatial and temporal regulation of the cloned promoters may be conducted by transcriptional fusion of the promoter fragments with a reporter gene such as the β-glucuronidase (GUS) reporter gene and transformation into a test plant such as tobacco.

Most preferably the first and second promoters are the same.

The first and second DNA sequences in the plant gene expression cassette of the invention may be on the same DNA construct or they may be on separate constructs which are used to co-transform a plant cell.

Preferably, the first and second DNA sequence are on the same construct, and are arranged in tandem. Thus in a particular embodiment, the invention provides a plant gene construct comprising elements (i) and (ii) as defined above, directly adjacent one another.

As illustrated hereinafter, transformation of plants using the plant gene expression cassettes of the invention results in a very large increase in carotenoid content, and therefore nutrient value of crops such as tomato. In particular, by over-expressing the *β-Chy* from *Capsicum annum* in tandem with *Arabidopsis β-Lcy* under the control of the fruit-specific *Pds* promoter in tomato (cv.Moneymaker), the level of β-carotene in the fruit was increased by 12-fold and the level of xanthophylls increased by 24-fold. Leaf carotenoid composition remained largely unaltered, presumably as a result of the use of the fruit-specific promoter. Over-expression of the *β-Chy* gene alone in the *B* genotype showed no alteration in xanthophyll levels, suggesting a post-translational control mechanism different from that acting in the Moneymaker line.

No significant differences in isomeric composition between the B line and most of the *β-Lcy* transformants were found. Both types of lines predominantly accumulated all-trans β-carotene. RT-PCR and Western blotting detected sufficient expression of *Capsicum* β-carotene hydroxylase, both at the mRNA and the protein levels in all transformed lines. Therefore, we are unable to state the precise reasons for the lack of xanthophyll production in the *B(Chy)* transformants. The proposed existence of carotenogenic enzymes as multi-enzyme complexes (Cunningham, F. X., et al. (1998) *Ann.Rev. Plant Physiol. Plant Mol. Biol.* 49, 557-583.) could be one of the reasons. Unexpected increases in β-carotene levels have been reported previously in rice and tomato when plants were transformed with the *Erwinia* phytoene desaturase. Protein-protein interactions between phytoene desaturase and lycopene β-cyclase could play a role (discussed in Giuliano, Get al. (2000) *Trends Plant. Sci.5,* 406-409). Similarly, a specific protein-protein interaction between the canonical lycopene β-cyclase and the β-carotene hydroxylase could be necessary for β-carotene hydroxylation. It is possible that this interaction does not occur between the endogenous *B* gene product and the introduced β-carotene hydroxylase.

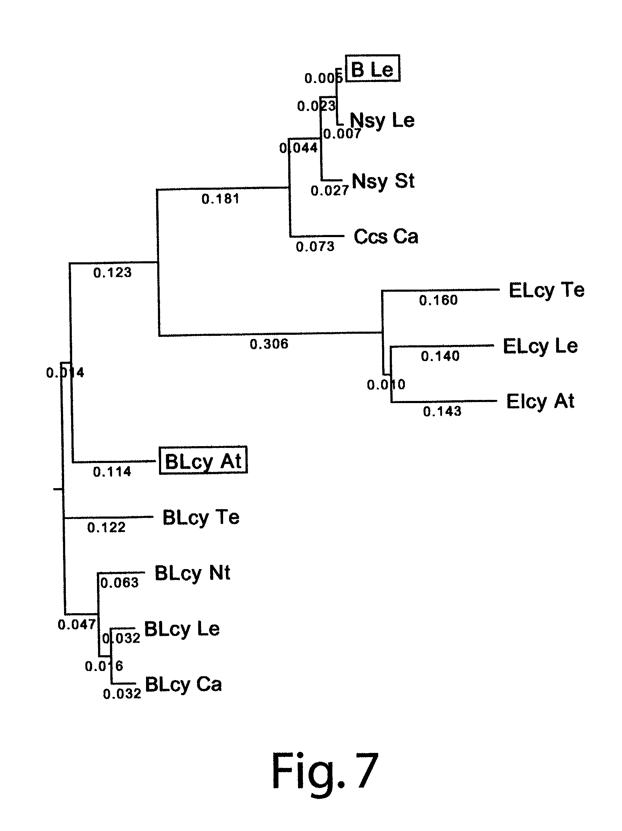

Indeed, the primary sequence of the *B* gene product is rather different from that of canonical β-cyclases (Figure 7). It is much more similar to that of enzymes such as capsanthin-capsorubin synthase (Ccs) and neoxanthin synthase (Nsy). In fact, the mature forms of tomato Nsy and B are almost identical, except for 3 amino acid differences. B and Ccs show some β-cyclase activity, while Nsy is devoid of such an activity. None of these enzymes has been shown convincingly to act *in vivo* upstream of β-hydroxylase.

It was also noted that the Moneymaker and the *B* line as well as all the transformant lines showed similar levels of expression of the endogenous lycopene β-cyclase and β-carotene hydroxylase genes. This suggests that the production of xanthophylls in the *MM(Lcy+Chy)* lines is more likely to be the result of the expression of the introduced transgenes rather than of a deregulation of endogenous genes.

The level of the increase is unexpected. Furthermore, the present invention is the first report of elevation of xanthophylls in ripe tomato fruits by metabolic engineering.

By increasing the carotenoid levels in the manner one describes, it may be expected that levels of carotenoid metabolites including water-soluble oxidation products, such as crocetin, or volatile molecules involved in flavour may be increased. Thus it may be possible to use the tomato as "cell factory" for carotenoids and carotenoid metabolites.

Thus in a further aspect the invention provides a process for increasing the metabolites of carotene content of a crop, said process comprising transforming a plant cell from which viable plants maybe recovered, with a plant gene expression cassette as described above, and thereafter generating viable plants from said cell.

In this context, "metabolites of carotene" include carotenoids such as those described above, and metabolites of these (β-carotene, zeaxanthin, astaxanthin).

Transformation of the plant cells may be effected using any of the conventional processes. In particular, processes such as *Agrobacterium* transformation, bombardment or via whiskers may be used, depending upon the particular plant species being transformed.

Suitably, the transformation is effected such that the plants are stably transformed, such that the feature is maintained in the progeny.

Plants and plant cells obtained using the process of the invention, and progeny and seeds thereof form a further aspect of the invention.

In particular, the plants will be, in addition to tomato, those crops mentioned above. They may suitably be oil-producing crops or other fruits, such as banana, rice, corn, wheat, fish food component, sunflower, canola, soya, cotton or flax. Marigolds also are used as a source of lutein and Lemna or pondweed have been suggested as sources of extractable materials and these plants also may be usefully transformed as described above. Tomato is a important crop in most areas of the globe, including developing countries. This invention illustrates the feasibility of synthesizing anti-oxidant xanthophylls in tomato fruits through the genetic engineering of appropriate genes.

The invention will now be particularly described only by way of Example with reference to the accompanying drawings in which:
Figure 1 illustrates diagrammatically the carotenoid metabolic pathway leading to β-carotene and xanthophyll synthesis. In this diagram, Psy = Phytoene synthase; Pds = Phytoene desaturase; Zds = ζ-carotene desaturase; β -and ε-Lcy = Lycopene β- and s- cyclases; β- and s- Chy = β- and ε-hydroxylases. [The existence of a separate ε-hydroxylase is hypothetical.]
Figure 2 is a schematic representation of the DNA constructs used in cassette of the invention.
Figure 3 shows the phenotypes of fruits (10 days after breaker stage) obtained from Moneymaker and *MM(Lcy +Chy )3* transformant (To) and its T1 progeny.
Figure 4 shows the RT-PCR analysis of the expression of *Arabidopsis β-Lcy* and *Capsicum β-Chy* in tomato fruits (10 days after breaker stage). The cDNA dilutions used were 1:10 (*E*), 1:5 (*CaChy*) and 1:20 (β-*Lcy*).
Figure 5 shows the Western analysis of *Arabidopsis* β-Lcy and *Capsicum* β-Chy proteins in fruit extracts from transformants.
Figure 6 shows the relative expression of endogenous carotenoid genes in the *MM(Lcy+Chy)* transformants (A) and the *B(Chy)* transformants (B) as detected by semi-quantitative RT-PCR in tomato fruits (10 days after breaker stage). Different cDNA dilutions (shown on the right) were amplified for 30 cycles (see Experimental Procedures) and the amplification products were separated on an agarose gel. The constitutive *E -1a* gene is also shown as an internal standard.
Figure 7 is a Clustal W (Thompson, J. D., Higgins, D. G. & Gibson, T. J. (1994) *Nucleic Acids Res* **22,** 4673-80). dendrogram, showing the relationships between different members of the plant carotene cyclase family. Abbreviations are: B = *B* gene product; Blcy = β-cyclase; Ccs = Capsanthin-capsorubin synthase; Elcy = ε-cyclase; Nsy = Neoxanthin synthase; At = *Arabidopsis thaliana;* Ca = *Capsicum annum;* Le = *Lycopersicon esculentum;* Nt = *Nicotiana tabacum;* St = *Solanum tuberosum;* Te = *Tagetes erecta.* The Arabidopsis β-Lcy and the tomato B are boxed.

### Example 1

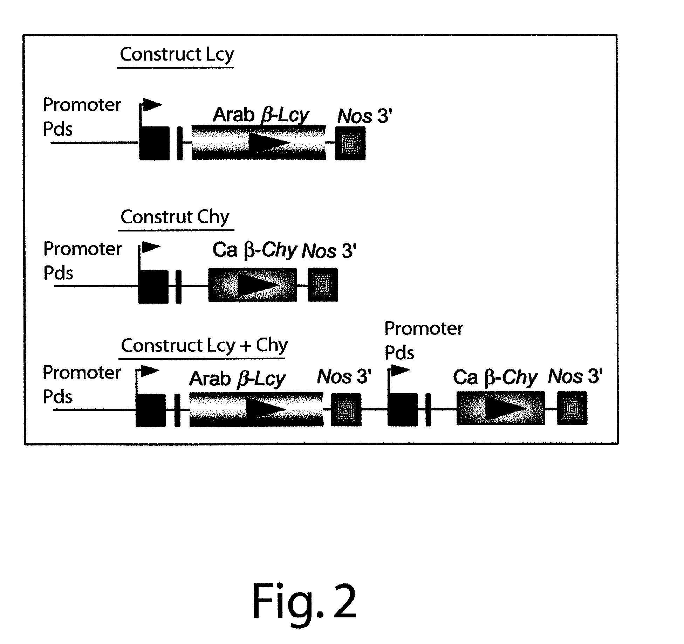

Three constructs were made to over-express the *Arabidopsis β-Lcy* and *Capsicum annum β -Chy* in tomato (Figure 2). Standard molecular biology protocols were followed as described (Sambrook, J., Fritsch, E. F., and Maniatis, T. (1989) *Moleclar Cloning. A Laboratory Manual (Second Edition),* Cold Spring Harbor Laboratory Press, Cold Spring Harbor).

The *Lcy* construct has been described previously (Rosati, C., et al. (2000) *Plant J.* 24, 413-420) and was made by fusing the *Arabidopsis β-Lcy* (Scolnik, P. A. et al., (1995) *Plant Physiol.* 108, 1343) to the tomato *Pds* promoter, which is up-regulated in tomato fruits during ripening (Corona, V., et al. (1996) *Plant J.* 9, 505-512).

The *Capsicum annum β-Chy* cDNA (Bouvier, F., et al. (1998) *Biochim. Biophys. Acta* 1391, 320-328) was fused separately to the *Pds* promoter to yield the *Chy* construct. A fragment of 1.1 Kb *Capsicum annum* carotene hydroxylase cDNA (Bouvier, F., et al. (1998) *Biochim. Biophys Acta* 1391, 320-328) was cloned as a *BamHI-Sacl* insert under the control of the *Pds* promoter in a pBI101-based transformation vector (Corona, V., et al. (1996) *supra*) which has a *Nos* terminator.

The *β-Lcy* construct - comprising the *Pds* promoter, the *Arabidopsis β-Lcy* (1.6 Kb) cDNA and the *Nos* terminator - was inserted next to the *Pds:: β-Chy* module, as a *Sall* fragment in the pBI-*CaChy - Nos* vector to make the double construct.

The transgenes were introduced into tomato plants according to the protocol of an Roekel *et al.* ((1993) *Plant Cell Rep.* 12, 644-647). All constructs were cloned between the right and left borders of the *Agrobacterium* T-DNA-based vector, pBI101, and introduced into tomato by Agrobacterium-mediated transformation. The *Lcy* and *Lcy+Chy* constructs were introduced in the cultivar Moneymaker *(MM)* whereas the *Chy* construct was introduced into the B line that carries the dominant *Beta* mutation leading to high β-carotene accumulation (Ronen, G., et al. (2000) *Proc. Natl. Acad. Sci. USA* 97, 11102-11107).

The primary transformants were grown and maintained in the greenhouse. Plants were grown in the greenhouse under controlled temperatures (15-25°C). Fruits were observed everyday for change of colour (breaker stage) and harvested 10 days later. The growth habit and appearance of the plants were comparable to the respective untransformed control plants (*MM* and *B).* PCR for presence of the transgenes was performed on leaf DNA of the transformants.

RT-PCR was performed as described (Giuliano, G., et al,.(1993) *Plant Cell* 5, 379-387) with some modifications. 600 ng of total RNA was retro-transcribed in a 30 ìl RT reaction volume containing 2.5µM of oligo-dT16. 2.5 µl (of different dilutions) of this mix was subjected to PCR using the oligonucleotides listed in Table 5. Each PCR cycle consisted of three steps of 45" each - a denaturation step at 94°C, an annealing step at 2°C below the Tm of the oligonucleotides and an elongation step at 72°C. 30 cycles were performed for all genes while adjusting the dilution to obtain a distinct, non-saturating signal. Each PCR was performed in triplicate and the quantified using a digital camera and 'NIH Image' software. Only PCR-positive plants were grown to maturity.

Frozen and lyophilized fruit pericarp were used for RNA isolation and HPLC analysis respectively. The protocol of Lichtenthaler ((1987) *Meth. Enzymol.* 148, 350-382) was used for spectrophotometric determination of leaf chlorophyll and carotenoids.

For HPLC analysis, the pigments were extracted successively with acetone and then with chloroform until total discoloration of the tissues. For red fruits the pigments were analyzed using the HPLC procedure described previously (Rosati, C., et al. (2000) *Plant J.* 24, 413-420). Otherwise, the pigment extract was subjected to HPLC analysis using a Zorbax ODS column (250 mm x 4.5 mm i.d.) eluted with 100% (v/v) solvent A (methanol/acetone/ water/ triethylamine, 0/17/11/0.1, v/v) for 10 min followed by a 5 min linear gradient to 100% solvent B (methanol/acetone, 60/40, v/v). β-carotene isomers were separated by HPLC using a Vydac C18 column as described previously (Quackenbush, F. W. (1987) *J. Liq. Chromatogr.* 10, 643-653). SDS/PAGE and Western blot analysis were performed as described (Suire, C.et al (2000) *PlantPhysiol.* 124, 971-978) using total fruit proteins extracted according to the hot phenol (Van Etten, J et al. (1979) *J. Bacteriol* 138, 650-652). In each case, 50 micrograms of proteins were used. The cyclase and the hydroxylase antibodies have been described previously (Bouvier F. et al., (1998) Biochim. Biophys. Acta., 1391, 320-328; Bouvier, F., et al. (1997) *Arch. Biochem. Biophys.* 346, 53-64) and were used at 1:2500 dilution. The cyclase antibodies reacted to a polypeptide of about 50 kD while the hydroxylase antibodies immunodetected a 20 kD polypeptide which correspond to the apparent mature forms of the two enzymes.

### Results

### Phenotypic analysis and transgene expression

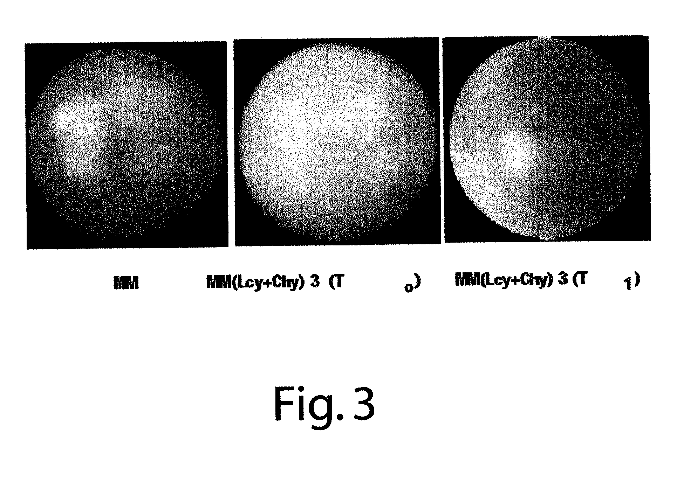

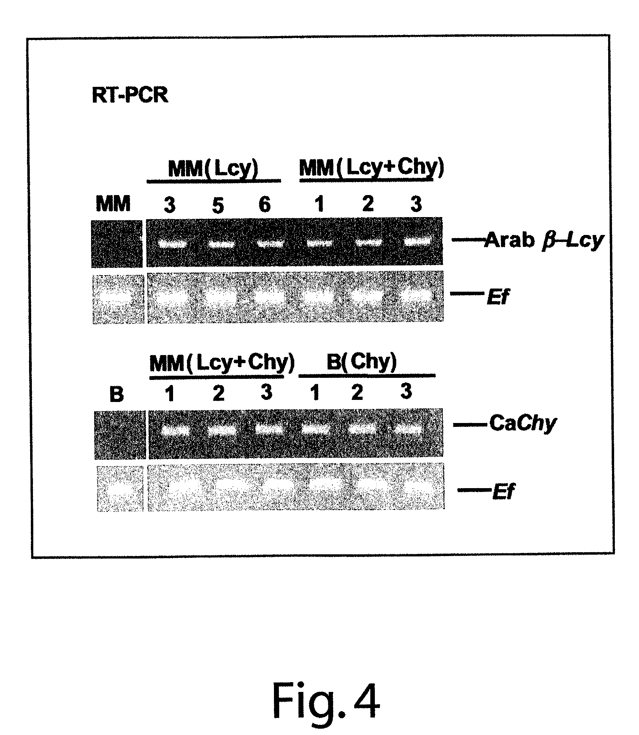

The analysis of three transgenic lines for each construct was completed. The color of the fruits varied from the complete red of wild type Moneymaker to red-orange to complete orange for several *MM(Lcy)* and *MM(Lcy+Chy)* transformants (Figure 3) indicating significant changes in the β-carotene/lycopene ratio. No changes in fruit phenotype were observed in the *B(Chy)* transformants when compared to the *B* parental line. The expression of the transgenes, *Arabidopsis β-Lcy* and *Capsicum β-Chy*, was detected by semi-quantitative RT-PCR (see Experimental Procedures). Figure 4 shows the relative expression of the two transgenes, compared to that of the housekeeping gene, *EF-1*α (Mahe, A et al., (1992) *Mol. Plant-Microbe Interact.* 5, 242-248). When compared to *EF-1α* the expression of *Arabidopsis β-Lcy* is approximately four-fold higher than that of *Capsicum β-Chy.*

### Carotenoid analysis

In fruits harvested 10 days after breaker stage, the *MM(Lcy)* transformants (3, 5 and 6) as well as the *MM(Lcy+Chy)* transformants (1, 2 and 3) show a 5-12 fold increase in β-carotene content over the parental Moneymaker line (Table 1). *MM(Lcy+Chy)3* produces as much as 63 µg gm⁻¹ fresh weight β-carotene compared to 5 µg gm⁻¹ for the *MM* line (Table 1 hereinafter). In most of the transformants, there is an increase in the total carotenoid content of the fruits (except in *MM(Lcy 6)* similar to that reported previously (Rosati, C., et al. (2000) *Plant J.* 24, 413-420).

As expected, the *B(Chy)* transformants show no major alterations in the β-carotene content when compared to the *B* line. To the opposite, the *MM(Lcy+Chy)* transformants 1, 2 and 3 show on an average a seven- and tenfold increase in the levels of the xanthophylls, primarily β-cryptoxanthin and zeaxanthin (Table 1). The total increase in xanthophylls is as much as 25 fold in *MM(Lcy+Chy)1.* It is very difficult to estimate the increase in β-cryptoxanthin + zeaxanthin since in untransformed fruits they are below the detection power of our analysis, but we estimate that it is more than 100-fold in all three transformants.

Surprisingly, no β-cryptoxanthin or zeaxanthin were detected in any of the *B(Chy)* transformants (Table 1) indicating that for some reason the overexpression of the *Capsium β-Chy* does not bring about the hydroxylation of the large pool of β-carotene present in *B* fruits.

In order to ascertain if the isomeric composition of the β-carotene present in B fruits could account for its unavailability to hydroxylation, the relative content of β-carotene isomers was analyzed by HPLC (Table 2) in fruits from *B* as well as that from *MM(β-Lcy)* transformants.

**Table 2.**

| Isomer composition (%) of high β-carotene expressing lines | | | |
|---|---|---|---|
| **Line** | ***Trans-β-* carotene** | **13*-cis*** *β-* **carotene** | **9*-cis*** *β-* **carotene** |
| B | 100 | ND* | ND* |
| | | | |
| Lcy (MM) 3 | 97 | ND* | ND* |
| Lcy (MM) 5 | 46 | 29 | 25 |
| Lcy (MM) 6 | 99 | ND* | ND* |

| | | | |
|---|---|---|---|
| *Not detected | | | |

The major β-carotene isomer in the B line is the all-trans configuration. The same isomer forms a large proportion of total β-carotene in the *MM(β-Lcy)* transformants. This suggests that: a) the overexpression of *Arabidopsis* β-Lcy causes the accumulation of *all-trans* β-carotene, which is then correctly hydroxylated by *Capsicum* β-Chy and b) the same isomer is present in the *B* line, but is somehow unavailable to hydroxylation by the same enzyme. The lack of hydroxylated β-carotene products in the *B(Chy)* transformants is therefore likely to be due to control exerted at the post-transcriptional level.

The leaf carotenoid and chlorophyll content for all the lines was analyzed spectrophotometrically (Lichtenthaler, H. (1987) *Meth. Enzymol.* 148, 350-382) and by HPLC. Table 3 (hereinafter) shows that the levels are essentially unchanged in all transformants. The *B* genotype has a higher carotenoid and chlorophyll content and this is also observed for all the *B(Chy)* transformants.

### Expression of the introduced proteins and of endogenous carotenoid genes

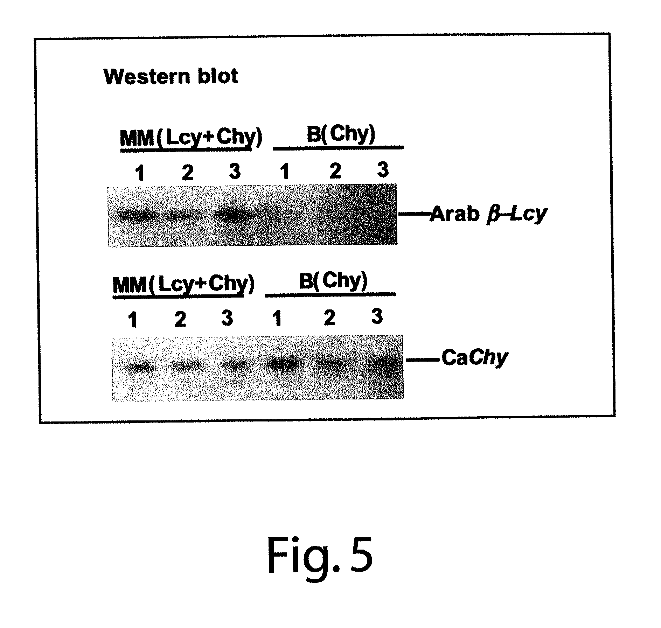

Western blotting was performed on total protein extracts using antibodies to the *Capsicum* β-Chy protein. Figure 5 shows that the protein is expressed, at detectable levels, in all the *MM(Lcy+Chy)* and *B(Chy)* transformants. In addition, antibodies raised against the *Capsicum β-Lcy* protein detected the expression of the Arabidopsis protein in all the *MM(Lcy+Chy)* transformants. Some expression of an endogenous protein cross-reacting with the anti-β-Lcy antibody and showing an similar apparent molecular weight to Arabidopsis β -Lcy is observed in the *B* line. We hypothesize that it is the *B* gene product (Ronen, G., et al. (2000) *Proc. Natl. Acad. Sci. USA* 97, 11102-11107). Taken together, these data suggest that a post-translational control mechanism is responsible for the inability of the expressed *Capsicum* β-Chy to produce xanthophylls in the *B* cells line despite the availability of large amounts of β-carotene in the all-trans configuration.

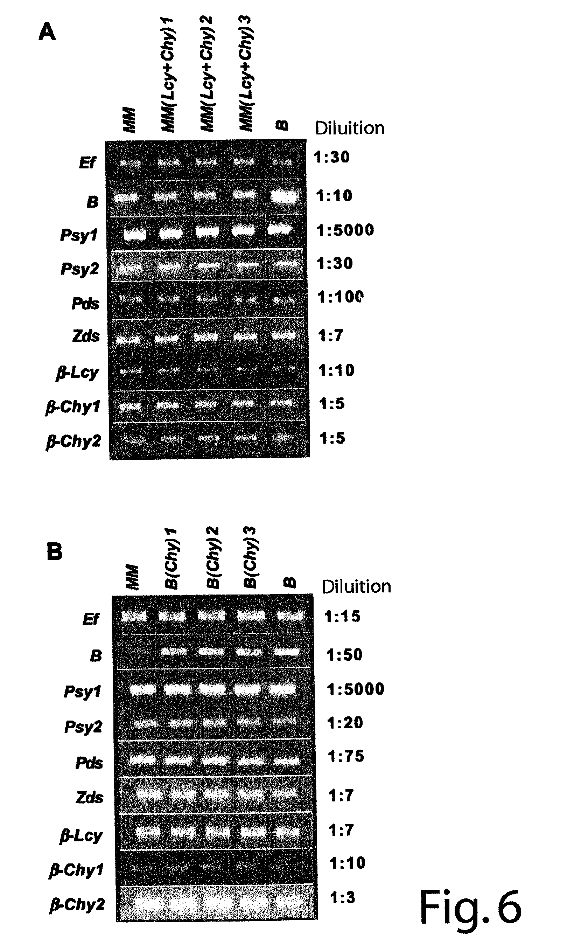

The expression of the endogenous genes for the whole carotenoid pathway from GGPP to zeaxanthin was measured by semi-quantitative RT-PCR (Figure 6). Apart from minor differences, the expression of all the carotenogenic genes was similar in all the lines. As expected, the expression of the *B* gene (Ronen, G., et al. (2000) *Proc. Natl. Acad. Sci. USA* 97, 11102-11107) was significantly higher in the *B* genotype. Figure 6 shows a comparison of the amounts of RT-PCR products obtained for each gene. A complete list of primers and conditions used for RT-PCR is provided in Table 5.

The inheritance of the transgenes was analyzed on leaf DNA by PCR. Table 4 shows that they are inherited in Mendelian fashion with a ratio of approximately 3:1.

**Table 4.**

| Inheritance of transgenes in the T1 generation (PCR-positive plants) | | |
|---|---|---|
| **Line** | ***AtLycBC*** | ***CaCHY*** |
| MM (Lcy + Chy) 2 | 10/12 | 10/12 |
| MM (Lcy + Chy) 3 | 10/12 | 10/12 |
| B (Chy) 2 | | 18/22 |
| B (Cht) 3 | | 21/27 |

The carotenoid content of fruits obtained from the progeny (Figure 3) of primary transformants was analyzed by HPLC and results similar to those shown in Table 1 were obtained, i.e. elevated levels of β-carotene in *MM(Lcy)* transformants and of β-carotene and xanthophylls in caseof the *MM(Lcy+Chy)* transformants (data not shown). Therefore, the phenotypes are heritable and co-segregate with the presence of the transgenes.

Other modifications of the present invention will be apparent to those skilled in the art without departing from the scope of the invention.

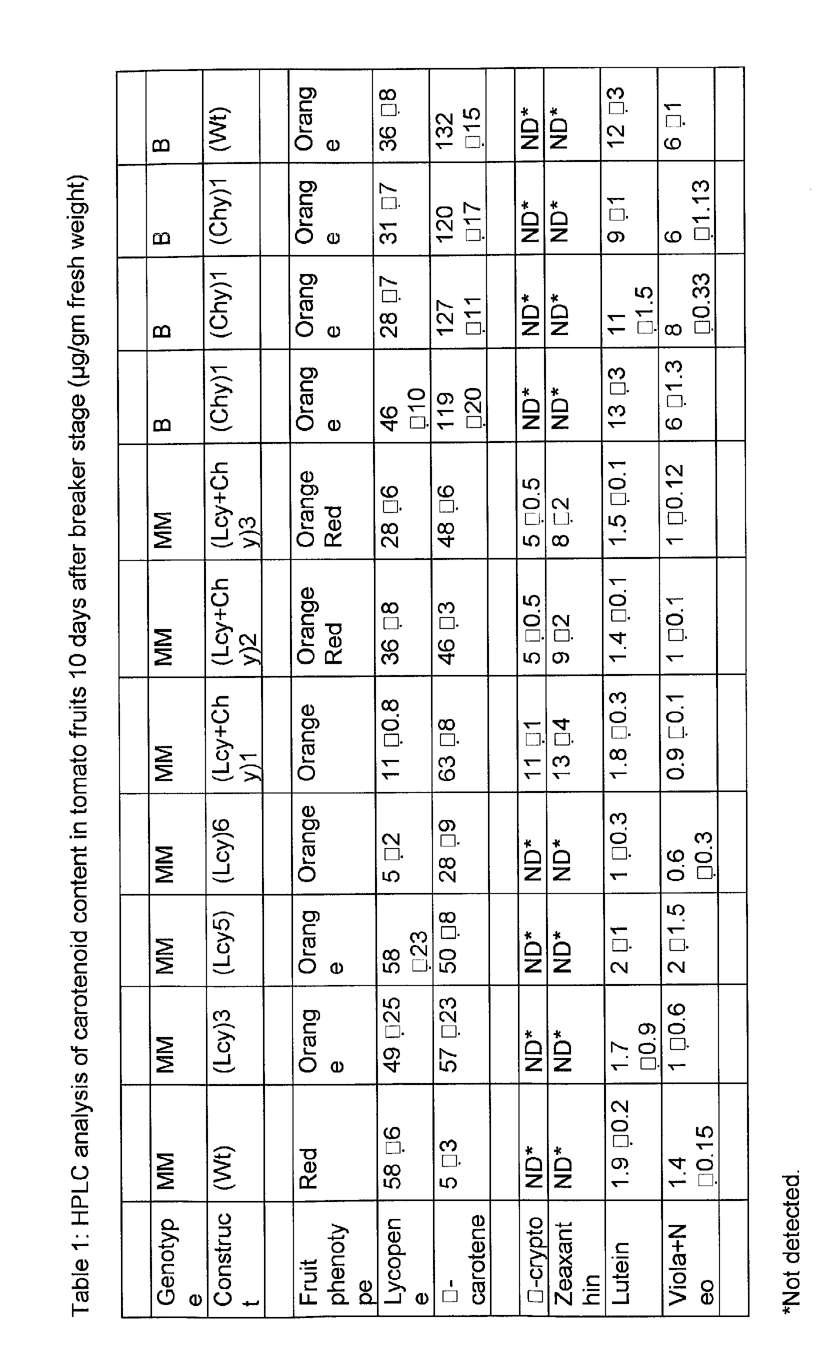

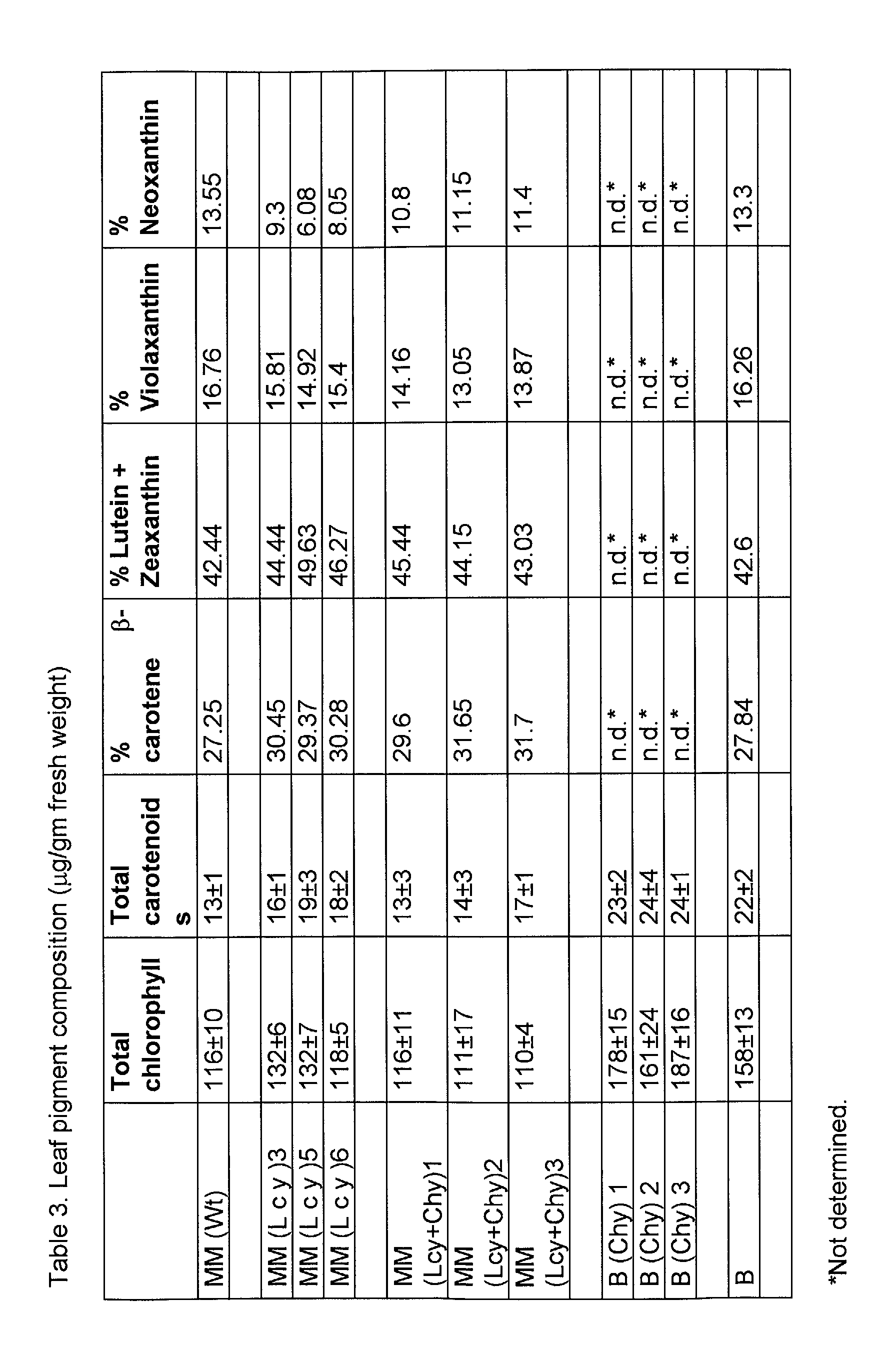

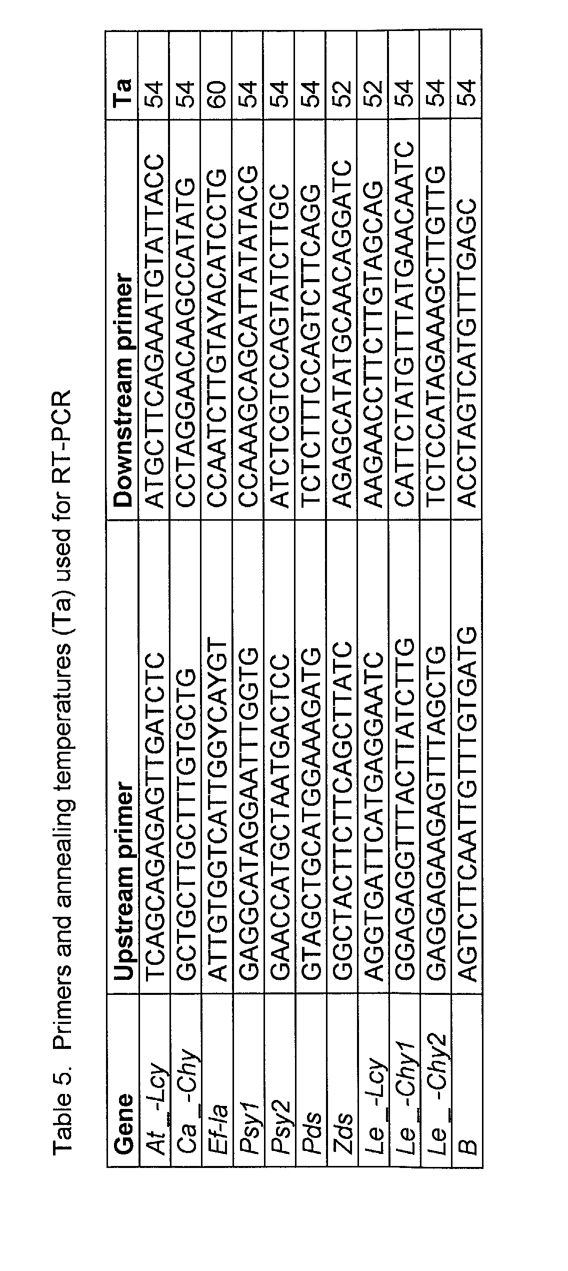

## Claims

1. A process for increasing the metabolites of carotene content of a plant, said process comprising upregulating at least a gene which encodes carotene hydroxylase activity.

2. A process according to claim 1 comprising upregulating a gene which encodes either lycopene β-cyclase and β-carotene hydroxylase activity.

3. A process according to claim 2 wherein upregulation is effected by transforming the plant so that the genome contains more copies of genes, which encode enzymes having lycopene β-cyclase and β-carotene hydroxylase activity.

4. A process according to claim 3 wherein both lycopene β-cyclase and a β carotene hydroxylase activity are upregulated in a plant.

5. A process according to any one of the preceding claims wherein the plant is transformed so that it contains additional copies of a gene encoding the said activity.

6. A plant gene expression cassette, which comprises a first DNA sequence which is capable of producing an over-expression of a gene which encodes a protein having lycopene β-cyclase activity and a second DNA sequence which is capable of producing an over-expression of a gene which encodes a protein having β-carotene hydroxylase activity.

7. A plant gene expression cassette according to claim 6 wherein the first DNA sequence comprises a sequence which encodes a protein having lycopene β-cyclase activity under the control of a plant promoter sequence.

8. A plant gene expression cassette according to claim 6 or claim 7 wherein the second DNA sequence comprises a sequence which encodes a protein having β-carotene hydroxylase activity under the control of a plant promoter sequence.

9. A plant gene expression cassette according to any one of claims from 6 to 8 wherein protein having lycopene β-cyclase activity is a wild-type enzyme or an active fragment thereof, or a variant of any of these.

10. A plant gene expression cassette according to any one of claims from 6 to 9 wherein the protein having β-carotene hydroxylase activity is a wild-type enzyme or an active fragment thereof, or a variant of any of these.

11. A plant gene expression cassette according to claim 9 or claim 10 wherein the protein is a wild-type enzyme.

12. A plant gene expression cassette according to any one of claims from 6 to 11 wherein the first DNA sequence is a gene which encodes a lycopene β-cyclase which is heterologous in comparison with that one of the plant to be transformed.

13. A plant gene expression cassette according to any one of claims from 6 to 12 wherein the second DNA sequence is a gene which encodes a β-carotene hydroxylase which is heterologous in comparison with that one of the plant to be transformed.

14. A plant gene expression cassette according to claim 6 which comprises
(i) a first DNA sequence which encodes a protein having lycopene β-cyclase activity under the control of a first plant promoter, and
(ii) a second DNA sequence which encodes a protein having β-carotene hydroxylase activity under the control of a second plant promoter.

15. A plant gene expression cassette according to claim 14 wherein the first and second plant promoters are promoters which are specific for a crop tissue of the plant.

16. A plant gene expression cassette according to claim 14 or claim 15 wherein the first and second promoters are the same.

17. A plant gene expression cassette according to any one of claims from 14 to 16 wherein (i) and (ii) are present on the same DNA construct.

18. A plant gene expression cassette according to claim 17 wherein (i) and (ii) are arranged in tandem.

19. A DNA construct comprising elements (i) and (ii) as defined in claim 14 directly adjacent one another.

20. A process for increasing the metabolites of carotene content of a plant, said process comprising transforming a plant cell from which viable plants may be recovered, using a plant gene expression cassette according to any one of claims from 6 to 18 or a DNA construct according to claim 19, and thereafter generating viable plants from said cell.

21. A plant or plant cell comprising a plant gene expression cassette according to any one of claims from 6 to 18 or a DNA construct according to claim 19.

22. Progeny and seeds of a plant according to claim 21 which comprises a plant gene expression cassette according to any one of claims from 6 to 18 or a DNA construct according to claim 19.

23. A plant or plant cell according to claim 21 or progeny according to claim 22 which is a tomato, banana, rice, corn, wheat, sunflower, canola, soya, cotton or flax.
